# EUROPÄISCHE PATENTSCHRIFT

(11) **EP 4 198 544 B1**
(45) Veröffentlichungstag und Bekanntmachung des Hinweises auf die Patenterteilung: **18.09.2024**
(21) Anmeldenummer: 21216119.4
(22) Anmeldetag: 20.12.2021
(51) Int. Cl.: G01R 33/28

(54) **INSTRUMENT, MAGNETRESONANZTOMOGRAPH UND VERFAHREN ZUM VERFOLGEN DES INSTRUMENTS**
INSTRUMENT, MAGNETIC RESONANCE TOMOGRAPH AND METHOD FOR TRACKING THE INSTRUMENT
INSTRUMENT, TOMOGRAPHE À RÉSONANCE MAGNÉTIQUE ET PROCÉDÉ DE SUIVI DE L'INSTRUMENT

(43) Veröffentlichungstag der Anmeldung: 21.06.2023
(73) Patentinhaber: Siemens Healthineers AG, 91301 Forchheim (DE)
(72) Erfinder: Schneider, Rainer, 91054 Erlangen (DE)
(74) Vertreter: Siemens Healthineers Patent Attorneys

(56) Entgegenhaltungen:
- EP-A1- 3 467 531
- DE-A1- 19 755 782
- US-A1- 2007 080 686
- US-A1- 2010 244 831
- US-A1- 2017 108 569

## Beschreibung

Die Erfindung betrifft ein Instrument, einen Magnetresonanztomographen sowie ein Verfahren zum Betrieb des Magnetresonanztomographen mit dem Instrument, bei dem die Relativposition des Instruments in Bezug auf den Magnetresonanztomographen ermittelt wird.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt. Zum Empfang des Signals werden vorzugsweise lokale Empfangsantennen, sogenannte Lokalspulen verwendet, die zur Erzielung eines besseren Signal-Rauschabstandes unmittelbar am Untersuchungsobjekt angeordnet werden.

Auf vorteilhafte Weise erlaubt ein Magnetresonanztomograph eine Visualisierung des Körperinneren über längere Zeit, ohne den Patienten oder Operateur einer erhöhten Dosis ionisierender Strahlung auszusetzen. Es sind jedoch Instrumente aus Kunststoff oder Metall durch die Magnetresonanztomographie nicht oder nur indirekt zu erfassen. Es sind beispielsweise zusätzliche Systeme mit Kameras und optischen Markern bekannt, die jedoch erheblichen Zusatzaufwand darstellen.

Aus dem Dokument DE 197 55 782 A1 ist eine Anordnung mit einem medizinischen Instrument zur Einführung in ein Untersuchungsobjekt und einer in oder an dem Instrument angebrachten Spulenanordnung mit mindestens einer Spule zum Empfangen und/oder Senden eines Hochfrequenzsignals, sowie ein Verfahren zur Positionsbestimmung eines solchen in ein Untersuchungsobjekt einführbaren medizinischen Instruments. Es wird vorgeschlagen, dass bei einer MR-Anordnung die Spulenanordnung mit einem Kondensator einen Resonanzkreis bildet und dass eine Modulationseinheit vorgesehen ist zum Modellieren eines in die Spulenanordnung eingekoppelten Hochfrequenzsignals.

Das Dokument US 2017/0108569 A1 beschreibt ein Magnetresonanzsystem. Das Magnetresonanzsystem ist ausgelegt, Magnetresonanzdaten aus einem Bildgebungsbereich zu erfassen. Es werden Magnetresonanz-Bilddaten mit aktiver Anregung durch einen Hochfrequenzpuls erfasst, gleichzeitig werden mit einer Hochfrequenzstörsignal-Erfassungsspule Hochfrequenzstörsignaldaten erfasst. Weiterhin werden Kalibrierungsdaten mit deaktivierter Anregung erfasst und gleichzeitig Hochfrequenz-Referenzdaten mit der Hochfrequenzstörsignal-Erfassungsspule. Aus den Hochfrequenz-Referenzdaten und den Kalibrierungsdaten wird eine Störsignal-Kalibrierung ermittelt.

Aus dem Dokument EP 3467531 A1 ist ein Magnetresonanztomograph bekannt, der eine erste Empfangsantenne zum Empfang eines Magnetresonanzsignals aus einem Patienten in einem Patiententunnel, eine zweite Empfangsantenne zum Empfang eines Signals mit der Larmorfrequenz des Magnetresonanzsignals, und einen Empfänger aufweist. Die zweite Empfangsantenne ist außerhalb oder in der Nähe einer Öffnung des Patiententunnels angeordnet. Der Empfänger steht in Signalverbindung mit der ersten Empfangsantenne und der zweiten Empfangsantenne und ist ausgelegt, ein mit der zweiten Empfangstenne empfangenes Störsignal in einem von der ersten Empfangsantenne empfangenen Magnetresonanzsignal zu unterdrücken.

Es ist eine Aufgabe der Erfindung, ein Verfahren und eine Vorrichtung bereitzustellen, die mit geringem zusätzlichem Aufwand eine Lokalisierung der Instrumente erlaubt.

Die Aufgabe wird durch ein erfindungsgemäßes Instrument nach Anspruch 1, einen erfindungsgemäßen Magnetresonanztomographen nach Anspruch 3 sowie ein erfindungsgemäßes Verfahren nach Anspruch 5 gelöst.

Das erfindungsgemäße Instrument ist für eine medizinische Untersuchung oder Eingriff unter Verwendung eines Magnetresonanztomographen vorgesehen. Dies kann beispielsweise eine Biopsienadel oder ein Katheter oder ein chirurgisches Instrument sein.

Das Instrument weist einen Transponder auf, der ausgelegt ist, ein Ortungssignal in einem Frequenzbereich des Magnetresonanztomographen zu emittieren. Das Emittieren des Ortungssignal kann auf das Aussenden eines zugeführten Signals, beispielsweise von dem Magnetresonanztomographen, durch eine Antenne wie eine induktive Antennenschleife oder eine elektrische Antenne begrenzt sein, aber auch die Erzeugung durch einen Oszillator und/oder Modulator in dem Transponder einschließen. Als Frequenzbereich des Magnetresonanztomographen wird eine Frequenz angesehen, die der Magnetresonanztomograph mit seinen Empfängern aufnehmen kann. Vorzugsweise handelt es sich dabei um eine Larmorfrequenz eines von dem Magnetresonanztomographen zu erfassenden Kernspins bzw. eine Frequenz, die in einem Frequenzbereich kleiner 100 kHz, 500 kHz, 1 MHz oder 2 MHz um die Larmorfrequenz herum liegt.

Denkbar sind auch mehrere Transponder an einem Instrument, vorzugsweise über die Ausdehnung des Instruments verteilt, beispielsweise um dessen Orientierung oder Kontur darstellen zu können. Eine Trennung der Signale der Mehrzahl an Transponder kann beispielsweise durch Verwendung unterschiedlicher Frequenzen oder nachfolgend erläuterter Kodierungen erfolgen.

Als Ortungssignal wird dabei ein Signal angesehen, das von den Empfängern des Magnetresonanztomographen empfangen werden kann und eine Auswertung zur Positionsbestimmung erlaubt. Dies betrifft unter anderem eine Amplitude des Ortungssignals, die die Empfänger nicht übersteuert, d.h. einen linearen Bereich der Empfänger nicht überschreitet und so eine getrennte Verarbeitung und spätere Trennung von Ortungssignal und Magnetresonanzsignal erlaubt.

Gleichzeitig können so auf vorteilhafte Weise unterschiedliche Abschwächungen erfasst und zur Positionsbestimmung genutzt werden.

Das Ortungssignal weist eine Kennzeichnung durch eine Kodierung auf, die eine Unterscheidung von Magnetresonanzsignalen erlaubt. Ebenso können unterschiedliche Kodierungen die Unterscheidung der Signale mehrerer Transponder ermöglichen. Unterschiedliche Möglichkeiten zur Kodierung sind nachfolgend in den Unteransprüchen angegeben.

Die Kodierung ist eine Modulation mit einem Pseudozufallscode. Unter Pseudozufallscode wird eine Signal- bzw. Bitfolge verstanden, die sich innerhalb einer vorbestimmten Periode nicht wiederholt und besonders zur Autokorrelation eines Referenzsignals mit einem daraus durch Übertragung abgeleiteten Signal geeignet ist und erlaubt, eine Phasenbeziehung bzw. Verzögerung durch diese Übertragung zu ermitteln.

Auf vorteilhafte Weise erleichtert der Pseudozufallscode das Erkennen des Ortungssignals durch Autokorrelation und dadurch die Trennung der Signale und Ermittlung von Signalverzögerungen. Bei mehreren Transpondern können unterschiedliche Pseudozufallscodes auch zur Trennung der Signale der unterschiedlichen Transponder dienen. Vorzugsweise sind die unterschiedlichen Pseudozufallscodes zueinander orthogonal, sodass durch Faltung mit einem Pseudozufallscode das jeweilige Transpondersignal selektiert werden kann.

Auf vorteilhafte Weise erlaubt es die Kennzeichnung des Ortungssignals durch die Kodierung, das Ortungssignal für die Auswertung von dem Magnetresonanzsignal und Störungen zu trennen und umgekehrt das Ortungssignal in einem von Lokalspulen empfangenen Magnetresonanzsignal zu unterdrücken, um Artefakte zu vermeiden.

Der erfindungsgemäße Magnetresonanztomograph weist eine Mehrzahl an Sensoren zur Erfassung eines Störsignals und eine Entstörvorrichtung zur Unterdrückung von Störsignalen in von den Lokalspulen empfangenen Magnetresonanzsignalen auf. Als Sensoren sind hierbei alle Antennen im weiteren Sinn zu verstehen, die ausgelegt sind, elektrische und/oder magnetische im bereits genannten Frequenzbereich zu erfassen und zur weiteren Verarbeitung an den Magnetresonanztomographen bzw. die Entstörvorrichtung weiterzuleiten. Die Sensoren können beispielsweise Induktionsschleifen oder elektrische Antennen aufweisen, Vorverstärker, Filter, aber auch A/D-Wandler. Vorzugsweise sind die Sensoren in unterschiedlichen vorbestimmten Positionen um den Patiententunnel bzw. das Field of View (FoV) angeordnet, um Störsignale aus unterschiedlichen Richtungen zu erfassen und zu unterscheiden. Vorzugsweise sind nicht alle Sensoren auf einer Geraden oder einer Ebene angeordnet, sondern dreidimensional im Raum verteilt, also zumindest einer der Sensoren ist außerhalb einer Ebene mit den anderen Sensoren angeordnet. Mit anderen Worten, die Sensoren spannen einen dreidimensionalen Raum auf.

Durch diese Anordnung der Sensoren ist es aber auf vorteilhafte Weise auch möglich, das Ortungssignal mit mehreren Sensoren zu erfassen und den Ursprungsort zu bestimmen bzw. zu triangulieren.

Die Entstörvorrichtung ist ausgelegt, ein Störsignal, d.h. ein Signal, das nicht von den zu untersuchenden Kernspins stammt und von den Lokalspulen bzw. der Körperspule gemeinsam mit dem Magnetresonanzsignal zusammen erfasst wird, in den Signalen der Lokalspule bzw. Körperspule in Abhängigkeit von den Signalen der Sensoren zu reduzieren. Dies kann beispielsweise erreicht werden, indem die Entstörvorrichtung die Signale mit einer Phasenverschiebung und mit einer Dämpfung versieht und von den Signalen der Lokalspulen bzw. der Körperspule subtrahiert. Dabei erlaubt es insbesondere die Kodierung, das Ortungssignal von den Magnetresonanzsignalen und anderen Störsignalen zu unterscheiden und damit zu trennen. Auf vorteilhafte Weise kann so auch das Ortungssignal als Störsignal interpretiert werden und dessen Auswirkungen auf das Magnetresonanzsignal und die damit erstellten Abbildungen reduziert werden.

Der Magnetresonanztomograph weist weiterhin eine Ortungsvorrichtung auf, die ausgelegt ist, mit den Sensoren ein Ortungssignal eines Transponders aufzunehmen. Es ist dabei beispielsweise denkbar, dass die Ortungsvorrichtung parallel zu der Entstörvorrichtung die Signale der Sensoren erhält und diese auswertet. Es ist aber beispielsweise auch denkbar, dass die Entstörvorrichtung bereits eine Quelltrennung vornimmt und so bereits der Ortungsvorrichtung Ortungssignalanteile der Sensoren ohne Magnetresonanzsignalanteile oder Störsignalanteile liefert. Dabei kann es auch sein, dass die Ortungsvorrichtung und die Entstörvorrichtung eine gemeinsame Hardwareplattform nutzen, beispielsweise auch als Softwaremodule auf gemeinsam genutzte Prozessoren ausgeführt werden.

Die Ortungsvorrichtung ist ausgelegt, eine Relativposition des Transponders relativ zu den Sensoren zu bestimmen. Denkbar ist es beispielsweise, dass unterschiedliche Amplituden der einzelnen Sensoren im Sinne des Abstandsgesetzes für elektromagnetische Felder aufgrund der Abschwächung einen Abstand des Transponders von dem jeweiligen Sensor angeben. Denkbar ist es auch, dass eine Phase der Signale und eine damit verbundene Signalverzögerung ebenfalls als Abstandsinformation genutzt wird. Dabei ist es auch denkbar, dass die Kodierung eine Phaseninformation liefert, die über die für die Trägerwelle auf eine ganze Periode begrenzte Zeit hinausgeht und damit größere Abstände und/oder höhere Frequenzen erlaubt.

Die Entstörvorrichtung kann beispielsweise diese unterschiedlichen Wege zwischen Transponder und Sensor zur Positionsbestimmung über Amplitude und Phase nutzen, um die Positionsermittlung durch Fehlerminimierung sicherer und genauer zu gestalten.

Denkbar sind auch neuronale Netzwerke zur Implementierung der Ortungsvorrichtung, die als Eingangswerte die Signale der Sensoren oder die bereits getrennten Signale von der Entstörvorrichtung erhält und aus diesen bei vorbestimmter Position der Sensoren die Koordinaten bzw. Relativposition ermittelt. Ein Training des neuronalen Netzwerkes ist dabei mit Erfassung der Sensordaten für vorbestimmte Positionen des Transponders im Patiententunnel oder FoV denkbar.

Ein neuronales Netzwerk ist auf vorteilhafte Weise in der Lage, auch komplexe Ausbreitungsbedingungen durch Reflexion im Patiententunnel zu berücksichtigen.

Auf vorteilhafte Weise erlauben es die Kodierung, die Sensoren und die Entstörvorrichtung in Zusammenarbeit mit der Ortungsvorrichtung, eine zuverlässige Lokalisierung des Transponders und damit des Instruments mit den vorhandenen Elementen des Magnetresonanztomographen zu ermöglichen, ohne die Bildqualität der Magnetresonanztomographie zu verschlechtern.

Das erfindungsgemäße Verfahren ist ein Verfahren zum Bestimmen der Relativposition eines medizinischen Instruments mit einem Magnetresonanztomographen relativ zu dem Magnetresonanztomographen. Das Instrument ist ein erfindungsgemäßes Instrument, wie es bereits vorhergehend beschrieben wurde. Der Magnetresonanztomograph ist ebenfalls ein erfindungsgemäßer Magnetresonanztomograph, wie er vorab beschrieben wurde.

In einem Schritt wird ein kodiertes Ortungssignal mit dem Transponder erzeugt. Dies kann auf unterschiedliche Arten geschehen. Der Transponder kann beispielsweise einen Oszillator aufweisen, der ein Hochfrequenzsignal mit einer Frequenz in dem bereits näher dargelegten Frequenzbereich des Magnetresonanztomographen erzeugt. Die Kodierung kann beispielsweise bereits in der Frequenz des Hochfrequenzsignals liegen, indem es zwar in dem von dem Magnetresonanztomographen empfangbaren Frequenzbereich liegt, gleichzeitig aber außerhalb der Frequenzen der Kernspins während der Bilderfassung liegt und somit die Bilderfassung nicht stört. Denkbar ist auch, dass der Transponder einen Modulator aufweist, mit dem dem Hochfrequenzsignal des Oszillators eine Kodierung aufmoduliert wird. Denkbare Kodierungen sind näher zu den Unteransprüchen erläutert. Mittels der Kodierung kann auf vorteilhafte Weise das Ortungssignal von Störsignalen und Magnetresonanzsignalen getrennt werden.

In einer Ausführungsform des erfindungsgemäßen Magnetresonanzsignals wird dem Transponder das Hochfrequenzsignal von dem Magnetresonanztomographen zugeführt, beispielsweise über eine Signalleitung oder drahtlos, anstatt es lokal durch einen Oszillator zu erzeugen. Denkbar ist auch, dass lokal durch Teiler, Vervielfacher oder PLL von dem zugeführten Hochfrequenzsignal das Ortungssignal abgeleitet wird. Dadurch kann eine definierte Frequenz- und/oder Phasen-Beziehung hergestellt werden, die zur Positionsbestimmung genutzt werden kann. Auch ist das Ortungssignal dann aufgrund der bekannten Parameter leichter durch die Entstörvorrichtung in einem von den Lokalspulen empfangenen Magnetresonanzsignal zu unterdrücken.

In einem weiteren Schritt des Verfahrens wird das Ortungssignal von dem Transponder über eine Antenne emittiert. Die Antenne kann beispielsweise eine induktive Antenne wie eine Induktionsschleife sein, oder eine elektrische Antenne wie ein Monopol oder Dipol. Denkbar ist, dass vorher die Amplitude des Ortungssignals durch ein Dämpfungsglied oder einen Verstärker in dem Transponder angepasst wird, sodass das Ortungssignal über die Sensoren oder Lokalspulen die Empfänger des Magnetresonanztomographen nicht übersteuert, vorzugsweise also im linearen Signalverarbeitungsbereich der Empfänger liegen.

In einem anderen Schritt des erfindungsgemäßen Verfahrens empfängt der Magnetresonanztomograph das Ortungssignal mit der Mehrzahl der Sensoren. Darunter ist zu verstehen, dass die Sensoren elektrische und/oder magnetische Wechselfelder des Ortungssignal erfassen und an den Magnetresonanztomographen weiterleiten. Dies kann auf analoge Weise nach einer optionalen Filterung und/oder Verstärkung in dem Sensor erfolgen. Das Weiterleiten kann analog erfolgen oder in digitaler Form. Das weitergeleitete Signal weist zumindest eine Information zu Amplitude, Frequenz und/oder Phase des Ortungssignals an den Sensoren auf, sodass aus dem Ortungssignal und der Position der Sensoren eine Position des Transponders ermittelt werden kann. Das Signal kann zur Auswertung an die Empfänger des Magnetresonanztomographen, die Entstörvorrichtung weitergeleitet werden, insbesondere aber wird es an die Ortungsvorrichtung weitergeleitet.

In einem weiteren Schritt trennt die Ortungsvorrichtung das von dem Transponder ausgesendete Ortungssignal von anderen Signalen wie Magnetresonanzsignal oder Störsignalen. Dies kann beispielsweise wie nachfolgend mit Hilfe der Kodierung erfolgen. Es ist aber auch denkbar, dass die Ortungsvorrichtung in Zusammenarbeit oder als Einheit mit der Entstörvorrichtung eine Quellentrennung der von den Sensoren und den Lokalspulen empfangenen Signalen vornimmt. Insbesondere kann mittels der Kodierung in den getrennten Signalen das Ortungssignal identifiziert werden.

Anschließend ermittelt die Ortungsvorrichtung eine Relativposition des Transponders relativ zu den Sensoren durch die von den Sensoren erfassten Ortungssignalen. Dazu wird auf die Ortungssignale eine Ortsbestimmungsrelation angewendet. Als Ortungsrelation wird hier eine Relation bezeichnet, die mit den von den Sensoren empfangenen Ortungssignal eine Relativposition des Transponders zu den Sensoren ermittelt. Dazu sind unterschiedliche Wege denkbar. Beispielsweise kann eine Dämpfung des Ortungssignals über die abstandsabhängige Dämpfung für elektromagnetische Felder zur Triangulation der Position des Transponders genutzt werden. Ebenso kann eine Zeitverzögerung, die durch eine Phasenverschiebung der Trägerwelle oder einer aufmodulierten Kodierung ermittelt werden kann, über die Laufzeit zur Triangulation dienen. Vorzugsweise werden diese unterschiedlichen Ansätze kombiniert, um im Sinne eines überbestimmten Systems die Fehler beispielsweise durch einen LSR-Algorithmus zu reduzieren. Denkbar ist hier auch als Implementierung der Ortsbestimmungsrelation ein neuronales Netzwerk, dass die optional wie bereits beschrieben vorbearbeiteten Signale der Sensoren als Eingangswerte erhält und als Ausgabewerte die Relativkoordinaten des Transponders liefert. Das Netzwerk kann dabei trainiert werden, indem für vorbestimmte Positionen des Transponders die Sensorsignale erfasst und dem Netzwerk zugeführt werden. Eine Abweichung der Ist-Position von der von dem neuronalen Netzwerk ermittelten Position kann dabei genutzt werden, um das Netzwerk z.B. durch Back-Propagation zu trainieren.

Das erfindungsgemäße Verfahren teilt die Vorteile des erfindungsgemäßen Magnetresonanztomographen. Darüber hinaus ermöglicht insbesondere ein neuronales Netzwerk eine zuverlässige Positionsermittlung in einer komplexen Umgebung mit vielfachen Reflexionen im Patiententunnel.

Weitere vorteilhafte Ausführungsformen sind zu den abhängigen Ansprüchen angegeben.

In einer möglichen Ausführungsform des erfindungsgemäßen Instruments kann der Oszillator beispielsweise einen Quarz-Oszillator oder einen auf andere Weise stabilisierten Hochfrequenzerzeuger aufweisen.

Auf vorteilhafte Weise kann so der Transponder ohne Leitungsverbindung ausgeführt werden und dessen Nutzung erleichtert werden.

Das mit dem erfindungsgemässen Instrument erzeugte Ortungssignal ist mittels der Frequenz sc kodiert, dass es zwar noch im Empfangsbereich der Empfänger des Magnetresonanztomographen ist, gleichzeitig aber nicht auf einer Frequenz, die das Magnetresonanzsignal während der Bilderfassung annimmt.

Auf vorteilhafte Weise kann so eine wechselseitige Störung von Bilderfassung und Ortung des Transponders reduziert werden.

In einer denkbaren Ausführungsform des erfindungsgemäßen Instruments ist der Transponder ausgelegt, das Ortungssignal von dem Magnetresonanztomograph zu erhalten und zu emittieren. Es ist dabei denkbar, dass das Ortungssignal oder vorzugsweise eine Grundfrequenz, von der das Ortungssignal durch Teiler, Vervielfacher oder PLL abgeleitet wird, über die Leitung oder drahtlos von dem Magnetresonanztomographen dem Transponder zugeführt wird.

So kann auf vorteilhafte Weise eine Störung des Magnetresonanztomographen durch das Oszillatorsignal verhindert und durch die feste Frequenz- und Phasenbeziehung eine Auswertung des Ortungssignals vereinfacht werden. Durch die feste Beziehung zwischen dem Ortungssignal und dem Signal, das vom Magnetresonanzsignals an den Transponder übertragen wird, kann das Ortungssignal anhand dieser Information auch leichter von der Entstörvorrichtung in den empfangenen Magnetresonanzsignalen unterdrückt werden.

Ein erfindungsgemäßes System weist ein erfindungsgemäßes Instrument und einen erfindungsgemäßen Magnetresonanztomographen auf. Auf vorteilhafte Weise ist die Ortungsvorrichtung des Magnetresonanztomographen ausgelegt, das Ortungssignal des Transponders des Instruments mittels der Kodierung von den Störsignalen zu trennen.

In einer Ausführungsform des erfindungsgemäßen Verfahrens weist dieses weiterhin den Schritt auf, den Transponder bzw. das Instrument mit dem Transponder an einer vorbestimmten Relativposition zu den Sensoren anzuordnen. Vorzugsweise ist diese vorbestimmte Relativposition in einem anwendungsgemäßen Arbeitsbereich des Instruments, vorzugsweise im FoV des Magnetresonanztomographen oder einem unmittelbar angrenzenden erweiterten Bereich, der noch von dem Magnetresonanztomographen abgebildet werden kann.

In einem weiteren Schritt wird die Ortsbestimmungsrelation mit Hilfe der vorbestimmten Relativposition und der ermittelten Relativposition kalibriert bzw. korrigiert, sodass das Ergebnis der Ortsbestimmungsrelation für das Ortungssignal des Transponders an der vorbestimmten Position weniger von der vorbestimmten Relativposition des Transponders abweicht.

Dabei ist es denkbar, dass die Schritte des Positionierens des Transponders an einer vorbestimmten Position und das anschließende Kalibrieren der Ortsbestimmungsrelation mit einer Mehrzahl an unterschiedlichen vorbestimmten Positionen, vorzugsweise über das FoV verteilt, wiederholt wird.

Auf vorteilhafte Weise kann so die Genauigkeit der Positionsbestimmung verbessert werden. Ist die Ortsbestimmungsrelation als neuronales Netzwerk implementiert, so kann auf diese Weise auch das neuronale Netzwerk trainiert werden.

In einer möglichen Ausführungsform des erfindungsgemäßen Verfahrens weist dieses den Schritt auf, eine Position des Instruments mit dem Transponder in einer Magnetresonanzaufnahme zu erfassen. Dies kann beispielsweise dadurch erfolgen, dass das Instrument bzw. der Transponder eine Markierung mit einer Magnetresonanz-aktiven Substanz oder einem auf der Larmorfrequenz aktivem Resonanzelement aufweist. Denkbar ist auch eine indirekte Abbildung des Instruments bzw. des Transponders, wenn diese Auswirkungen auf die Abbildung der Umgebung verursachen.

In einem weiteren Schritt wird dann die Magnetresonanzaufnahme in Abhängigkeit von der in der Magnetresonanzaufnahme erfassten Position des Transponders und der mit der Ortungsvorrichtung erfassten Position des Transponders kalibriert bzw. korrigiert.

Auf vorteilhafte Weise ist eine Positionsbestimmung durch das Ortungssignal unabhängig von Fehlern in der Homogenität des statischen Magnetfeldes oder Verzerrungen durch Wirbelströme und bietet so eine davon unabhängige Korrekturmöglichkeit.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- Fig. 1: eine schematische Darstellung eines erfindungsgemäßen Magnetresonanztomographen mit einem erfindungsgemäßen Instrument;
- Fig. 2: eine schematische Darstellung einer Hochfrequenzeinheit eines erfindungsgemäßen Magnetresonanztomographen;
- Fig. 3: eine schematische Darstellung eines erfindungsgemäßen Instruments;
- Fig. 4: ein schematisches Ablaufdiagramm einer Ausführungsform eines erfindungsgemäßen Verfahrens.

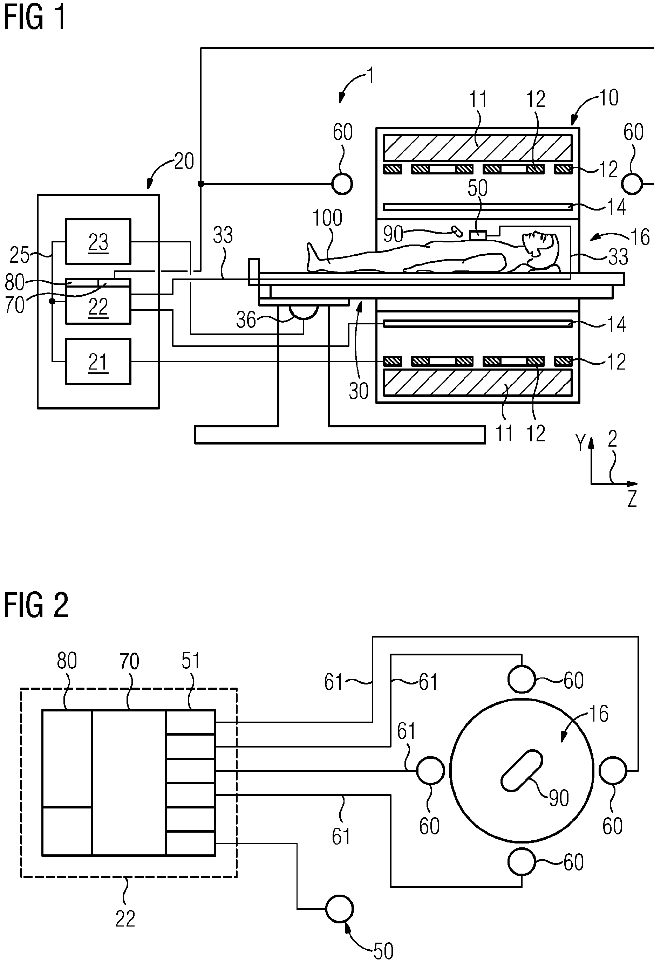

Fig. 1 zeigt eine schematische Darstellung einer Ausführungsform eines Magnetresonanztomographen 1 zum Ausführen des erfindungsgemäßen Verfahrens.

Die Magneteinheit 10 weist einen Feldmagneten 11 auf, der ein statisches Magnetfeld B0 zur Ausrichtung von Kernspins von Proben bzw. des Patienten 100 in einem Aufnahmebereich erzeugt. Der Aufnahmebereich zeichnet sich durch ein äußerst homogenes statisches Magnetfeld B0 aus, wobei die Homogenität insbesondere die Magnetfeldstärke bzw. den Betrag betrifft. Der Aufnahmebereich ist nahezu kugelförmig und in einem Patiententunnel 16 angeordnet, der sich in einer Längsrichtung 2 durch die Magneteinheit 10 erstreckt. Eine Patientenliege 30 ist in dem Patiententunnel 16 von der Verfahreinheit 36 bewegbar. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Magnetfeldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 zeitlich und räumlich variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu ausgelegt ist, ein über eine Signalleitung zugeführtes Hochfrequenzsignal in das Untersuchungsvolumen abzustrahlen und von dem Patient 100 emittierte Resonanzsignale zu empfangen und über eine Signalleitung abzugeben.

Eine Steuereinheit 20 versorgt die Magneteinheit 10 mit den verschiedenen Signalen für die Gradientenspulen 12 und die Körperspule 14 und wertet die empfangenen Signale aus.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 100 zu erzeugen. Dabei können Pulsleistungen im Bereich von Kilowatt erreicht werden. Die Anregungssignale können über die Körperspule 14 oder auch über eine lokale Sendeantenne in den Patienten 100 abgestrahlt werden.

Eine Steuerung 23 kommuniziert über einen Signalbus 25 mit der Gradientensteuerung 21 und der Hochfrequenzeinheit 22.

Sensoren 60 sind mit einer Entstörvorrichtung 70 verbunden. Die Sensoren 60 sind vorzugsweise in unterschiedlichen Raumrichtungen relativ zu dem Field of View des Magnetresonanztomographen 1 angeordnet, sodass die Sensoren 60 ein dreidimensionales Koordinatensystem aufspannen. Beispielsweise können, wie dargestellt, drei oder mehr Sensoren 60 entlang des Umfangs der Öffnungen des Patiententunnels verteilt sein. Vorzugsweise befinden sich Sensoren 60 an beiden gegenüberliegenden Öffnungen des Patiententunnels 16, um eine bessere Ortsbestimmung entlang der z-Achse zwischen den Öffnungen zu erlauben. Die Sensoren 60 sind beispielsweise Induktionsschleifen, die magnetische Wechselfelder erfassen und über Signalverbindungen 61 an die Hochfrequenzeinheit 22 weiterleiten. In der Hochfrequenzeinheit 22 sind eine Entstörvorrichtung 70 und Ortungsvorrichtung 80 vorgesehen, die die Signale der Sensoren 60 empfangen und verarbeiten.

Die Entstörvorrichtung 70 und die Ortungseinrichtung 80 können dabei auf einer gemeinsamen Hardware, beispielsweise einem Signalprozessor als Softwaremodul oder einem FPGA implementiert sein. Es ist aber auch denkbar, dass die Entstörvorrichtung 70 und die Ortungsvorrichtung 80 nur Teile der Signalverarbeitung gemeinsam haben oder ganz getrennt ausgelegt sind. Details zur Entstörvorrichtung 70 und der Ortungseinrichtung 80 sind nachfolgend zu Fig. 2 näher ausgeführt.

Ein erfindungsgemäßes Instrument 90 befindet sich im Patiententunnel und sendet dort mittels des Transponders 92 ein Ortungssignal aus, das von den Sensoren 60 erfasst wird.

Fig. 2 zeigt eine schematische Darstellung einer Hochfrequenzeinheit 22 eines erfindungsgemäßen Magnetresonanztomographen 1 mit einer Ortungsvorrichtung 80 und einer Entstörvorrichtung 70 sowie den Sensoren 60 und dem Instrument 90. Gleiche Gegenstände sind mit gleichen Referenzzeichen versehen.

Die Sensoren 60 sind um die Öffnung des Patiententunnels 16 herum angeordnet und empfangen dort das Ortungssignal des Instruments 90 bzw. dessen Transponders 92. Die Signale der Sensoren 60 werden über die Signalverbindungen 61 zu der Hochfrequenzeinheit 22 weitergeleitet. Dort wird durch Empfänger 51 das Signal aufbereitet, beispielsweise verstärkt, gefiltert und, sofern nicht bereits im Sensor 60 geschehen, auch digitalisiert. Bei den Empfängern 51 handelt es sich vorzugsweise um Empfänger, die auch zur Aufbereitung der Signale der Lokalspulen 50 zur Bilderfassung genutzt werden. Die vorbehandelten Signale der Sensoren 60 werden von den Empfängern 51 der Ortungsvorrichtung 80 und/oder der Entstörvorrichtung 70 zugeführt.

Die Entstörvorrichtung 70 ist dabei ausgelegt, mittels der Sensoren 60 elektromagnetische Störungen zu erfassen und durch destruktive Interferenz in den von der Lokalspule 50 erfassten Magnetresonanzsignalen zu reduzieren. Dazu kann unter anderem ein Quellentrennungsalgorithmus genutzt werden, der dann auch die Signale der Sensoren 60 nach den unterschiedlichen Quellen trennen kann, wie beispielsweise dem Ortungssignal und den Störquellen, was die nachfolgende Auswertung des Ortungssignals erleichtert. Auch stellt das Ortungssignal des Transponders 92 möglicherweise ein Störsignal für das Magnetresonanzsignal dar, sodass die Entstörvorrichtung 70 auf vorteilhafte Weise ein durch die Ortungsvorrichtung 80 erkanntes Ortungssignal genutzt werden kann, um das Ortungssignal mittels der Entstörvorrichtung 70 in den von der Lokalspule 50 erfassten Magnetresonanzsignalen besser zu unterdrücken.

Die Ortungsvorrichtung 80 kann dabei die Signale der Sensoren 60 auf unterschiedliche Weise auswerten. Beispielsweise können Phasenunterschiede zwischen den einzelnen Signalen als Laufzeitunterschiede und damit Abstände der Sensoren 60 zu dem Transponder 92 ausgewertet werden. Denkbar ist in Abhängigkeit von der Frequenz des Ortungssignal auch die Phasenverschiebung einer aufmodulierten Kodierung anstatt der Trägerwelle selbst, um Laufzeiten größer einer Periode der Frequenz des Ortungssignals erfassen zu können. Als Kodierung bieten sich beispielsweise Pseudozufallsfolgen an, die durch Kreuz- bzw. Autokorrelation mittels eines Korrelators in der Ortungsvorrichtung ausgewertet werden können. Auch ermöglicht es eine Pseudozufallsfolge auf vorteilhafte Weise, ein Signal unterhalb der Rauschgrenze mit dem Korrelator zu erfassen und auszuwerten und so die Störungen des Magnetresonanzsignals zu minimieren.

Auch kann eine unterschiedliche Amplitude der Sensorsignale als abstandsabhängige Abschwächung elektrischer und/oder magnetischer Felder in einen Abstandswert umgesetzt werden. Aufgrund der ermittelten Abstände des Transponders 92 zu den Sensoren 60 kann dessen Position ermittelt werden. Der Fehler kann insbesondere reduziert werden, wenn der Transponder 92 zwischen den Sensoren 60 angeordnet ist, also von einem Polygon mit Sensoren 60 auf den Eckpunkten eingeschlossen wird.

Die Ortsbestimmung kann durch eine Ortsbestimmungsrelation erfolgen, die als Eingangswerte die von den Sensoren 60 empfangenen Ortungssignale erhält und als Ausgangswert die Koordinaten der Relativposition des Transponders 92 zu den Sensoren 60 und damit zu dem Magnetresonanztomographen 1. Mit 4 Sensoren ist eine analytische Lösung denkbar. Eine größere Anzahl an Sensoren 60 liefert ein überbestimmtes System, das beispielsweise ein Optimierungsproblem darstellt, welches mittels eines Fehlerminimierungsverfahrens wie Least Square Root (LSR) eine genauere und zuverlässigere Positionsbestimmung ermöglicht. Auch können unterschiedliche Ergebnisse aus Amplitude und Laufzeit in diesem überbestimmten System genutzt werden, um die Positionsbestimmung weiter zu verbessern.

Es ist dabei auch denkbar, dass die Ortsbestimmungsrelation durch ein neuronales Netzwerk implementiert ist. Dabei kann das neuronale Netzwerk mit Trainingsdaten trainiert werden, die gewonnen werden, indem die Signale der Sensoren 60 für jeweils vorbestimmte Trainingspositionen des Transponders 92 erfasst werden. Die von dem neuronalen Netzwerk aus diesen Sensorsignalen bestimmten Relativpositionen werden mit den vorbestimmten Trainingspositionen verglichen und die Abweichungen mittels Back-Propagation reduziert.

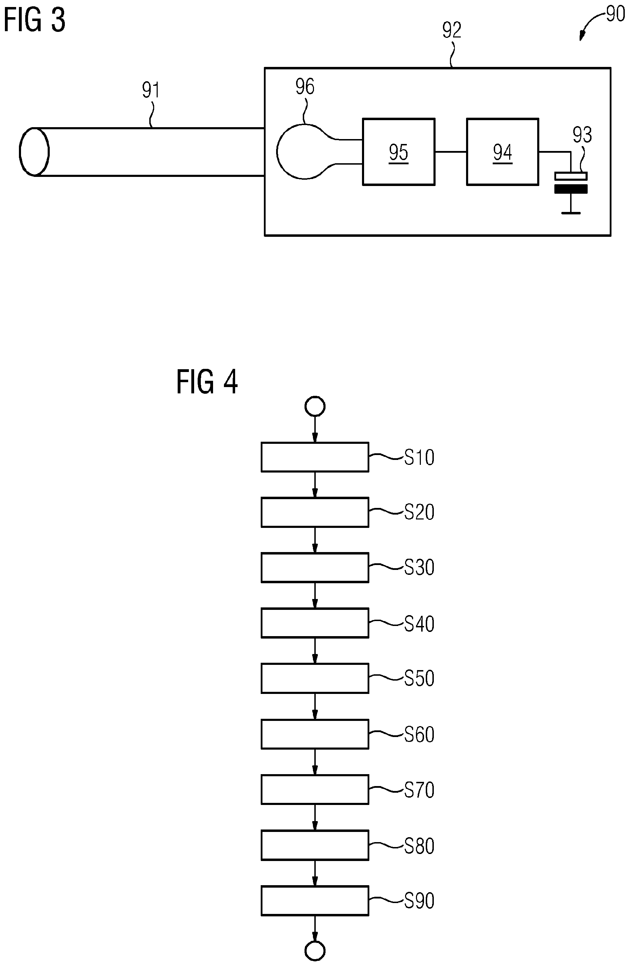

Fig. 3 stellt eine mögliche Ausführungsform eines erfindungsgemäßen Instruments dar. An einem chirurgischen Instrument wie beispielsweise einer Biopsienadel 91 ist ein Transponder 92 angeordnet. Der Transponder 92 weist eine Energiequelle 93 auf, beispielsweise eine Batterie oder einen wiederaufladbaren Akkumulator. Denkbar ist auch eine drahtlose Energieversorgung über Induktion. Die Energiequelle 93 versorgt die Komponenten des Transponders 92 mit Energie.

Ein Oszillator 94 erzeugt einen hochfrequenten Wechselstrom, dessen Frequenz vorzugsweise in einem Empfangsbereich der Empfänger des Magnetresonanztomographen 1 für das Magnetresonanzsignal liegt. Der hochfrequente Wechselstrom wird von einem Modulator 95 mit einer Kodierung moduliert, die ausgelegt ist, das Ortungssignal unterscheidbar von Magnetresonanzsignalen und/oder Störsignalen zu machen. Es ist auch denkbar, dass die Kodierung eine Zeitinformation zur Laufzeiterfassung umfasst. Das Ortungssignal wird anschließend von einer Sendeantenne 96 emittiert, beispielsweise einer Induktionsspule. Die Kodierung ist eine Modulation mit einem Pseudozufallscode. Unter Pseudozufallscode wird eine Signal- bzw. Bitfolge verstanden, die sich innerhalb einer vorbestimmten Periode nicht wiederholt und besonders zur Autokorrelation eines Referenzsignals mit einem daraus durch Übertragung abgeleiteten Signal geeignet ist und erlaubt, eine Phasenbeziehung bzw. Verzögerung durch diese Übertragung zu ermitteln.

Es ist in einer nicht durch die Ansprüche geschützten Ausführungsform des Instruments auch denkbar, dass der Transponder 92 das Hochfrequenzsignal nicht unabhängig erzeugt, sondern von einem Signal abgeleitet wird, das der Magnetresonanztomograph 1 über eine drahtgebundene oder eine drahtlose Verbindung zu dem Transponder 92 überträgt. Das Ortungssignal kann daraus beispielsweise durch einen Frequenzvervielfacher, Frequenzteiler oder PLL erzeugt werden. Auf vorteilhafte Weise kann so eine feste Frequenz- und Phasenbeziehung zu den Magnetresonanzsignalen sichergestellt werden, die eine störende Wechselwirkung zu den Magnetresonanzsignalen reduzieren lässt. Auch ist die Wirkung der Entstörvorrichtung 70 besser, wenn wie bei einem von dem Magnetresonanztomographen 1 erzeugten Ortungssignals die Eigenschaften genau bekannt sind.

Fig. 4 zeigt ein schematisches Ablaufdiagramm einer Ausführungsform eines erfindungsgemäßen Verfahrens.
In einem Schritt S20 wird ein kodiertes Ortungssignal mit dem Transponder 92 erzeugt, wie es bereits zu Fig. 3 erläutert wurde.

In einem Schritt S30 wird das kodierte Ortungssignals mit dem Transponder 92 ausgesendet, beispielsweise über dessen Sendeantenne 96.

Das von dem Transponder emittierte Ortungssignal wird in einem Schritt S40 mit der Mehrzahl der Sensoren 60 erfasst bzw. empfangen. Das Empfangen kann dabei auch eine Vorverarbeitung der empfangenen Signale durch Verstärker, Filter, Frequenzumsetzer und/oder A/D-Wandler umfassen. Ein Teil der Vorverarbeitung kann dabei auch in den Empfängern 51 des Magnetresonanztomographen 1 erfolgen.

In einem anderen Schritt S50 wir das Ortungssignals von weiteren durch die Sensoren 60 empfangenen Signalen durch die Ortungsvorrichtung 80 und/oder Entstörvorrichtung 70 getrennt.

In einem weiteren Schritt S60 wird eine Relativposition des Transponders 92 relativ zu den Sensoren 60 durch die Ortungsvorrichtung 80 mittels des von den Sensoren 60 erfassten Ortungssignals und einer darauf angewendeten Ortsbestimmungsrelation bestimmt. Denkbar ist beispielsweise die Nutzung einer Abschwächung oder Laufzeit des Ortungssignal zur Abstandsbestimmung von Transponder 92 und den jeweiligen Sensoren 60. Ein Prozessor der Ortungsvorrichtung 60 kann daraus beispielsweise über ein Optimierungsproblem eine Relativposition des Sensors mit möglichst kleinem Fehler ermitteln. Denkbar ist auch die Implementierung der Ortsbestimmungsrelation durch ein neuronales Netzwerk.

In einer denkbaren Ausführungsform des erfindungsgemäßen Verfahrens wird die Ortungsvorrichtung kalibriert bzw. das neuronale Netzwerk trainiert, indem in einem Schritt S10 der Transponder 92 an einer vorbestimmten Relativposition zu den Sensoren 60 positioniert wird. Gemäß der Schritte S20 bis S60 wird eine Relativposition des Transponders 92 mit der Ortungsvorrichtung 80 ermittelt und in einem Schritt S70 die Ortsbestimmungsrelation mit Hilfe der vorbestimmten Relativposition und der ermittelten Relativposition kalibriert bzw. trainiert.

In einer denkbaren Ausführungsform des erfindungsgemäßen Verfahrens wird eine Magnetresonanzaufnahme mit Hilfe der Ortungsvorrichtung kalibriert. In einem Schritt S80 wird eine Position des Instruments 90 mit dem Transponder 92 in einer Magnetresonanzaufnahme erfasst. Denkbar ist es dabei, dass das Instrument 90 einen Marker aufweist, der in einer Magnetresonanzaufnahme sichtbar wird, beispielsweise eine aktive Substanz oder einen elektrischen Resonator auf der Larmorfrequenz. Denkbar ist auch die Abbildung durch Artefakte, die das Instrument in einer Umgebung erzeugt.

Anschließend wird in einem Schritt S90 die Magnetresonanzaufnahme bzw. die Bildrekonstruktion des Magnetresonanztomographen durch Vergleich der mit der Ortungsvorrichtung erfassten Relativposition aus den Schritten S20 bis S60 mit der Position in der Magnetresonanzaufnahme die Magnetresonanzaufnahme kalibriert bzw. korrigiert.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Instrument für eine medizinische Untersuchung oder Eingriff unter Verwendung eines Magnetresonanztomographen (1), wobei das Instrument (90) mindestens einen Transponder (92) aufweist, der ausgelegt ist, ein Ortungssignal in einem Frequenzbereich des Magnetresonanztomographen (1) zu emittieren,
wobei das Ortungssignal eine Kodierung zur Unterscheidung von einem Magnetresonanzsignal aufweist,
wobei der Transponder (92) eine Energiequelle (93) und einen Oszillator (94) aufweist, wobei der Oszillator (94) ausgelegt ist, das Ortungssignal zu erzeugen,
**dadurch gekennzeichnet, dass**
der Transponder (92) einen Modulator (95) aufweist, die Kodierung eine Modulation mit einem Pseudozufallscode ist und der Modulator (95) ausgelegt ist, das Ortungssignal mit der Kodierung zu modulieren.

2. Instrument nach Anspruch 1, wobei die Kodierung eine Frequenzkodierung in einem Frequenzbereich des Magnetresonanztomographen (1) ist.

3. Magnetresonanztomograph, wobei der Magnetresonanztomograph (1) eine Mehrzahl an Sensoren (60) zur Erfassung eines Störsignals und eine Entstörvorrichtung (70) zur Unterdrückung von Störsignalen in von Lokalspulen empfangenen Magnetresonanzsignalen aufweist,
**dadurch gekennzeichnet, dass**
der Magnetresonanztomograph eine Ortungsvorrichtung (80) aufweist, die ausgelegt ist, mit den Sensoren (60) ein Ortungssignal eines Transponders (92) aufzunehmen und damit eine Relativposition des Transponders (92) relativ zu den Sensoren (60) zu bestimmen,
wobei das Ortungssignal eine Kodierung zur Unterscheidung von einem Magnetresonanzsignal aufweist und die Ortungsvorrichtung (80) des Magnetresonanztomographen (1) ausgelegt ist,
das Ortungssignal des Transponders (92) eines Instruments (90) mittels der Kodierung von den Störsignalen zu trennen.

4. System aus einem Instrument für eine medizinische Untersuchung oder einen medizinischen Eingriff unter Verwendung eines Magnetresonanztomographen (1), wobei das Instrument (90) mindestens einen Transponder (92) aufweist, der ausgelegt ist, ein Ortungssignal in einem Frequenzbereich des Magnetresonanztomographen (1) zu emittieren,
wobei das Ortungssignal eine Kodierung zur Unterscheidung von einem Magnetresonanzsignal aufweist
und einem Magnetresonanztomographen (1) nach Anspruch 3,
wobei der Transponder (92) ausgelegt ist, ein Ortungssignal mit einer Kodierung zu erzeugen oder der Magnetresonanztomograph (1) ausgelegt ist, ein Ortungssignal mit einer Kodierung zu erzeugen und über eine Leitung oder drahtlos von dem Magnetresonanztomographen dem Transponder zu übertragen
und die Ortungsvorrichtung (80) ausgelegt ist, das Ortungssignal des Transponders (92) mittels der Kodierung von den Störsignalen zu trennen.

5. Verfahren zum Bestimmen der Relativposition eines medizinischen Instruments (90) mit einem Magnetresonanztomographen (1), wobei das Instrument einen Transponder (92) zum Aussenden eines Ortungssignals aufweist, der Magnetresonanztomograph (1) eine Mehrzahl an Sensoren (60) zum Erfassen des Ortungssignals aufweist, wobei die Sensoren (60) in unterschiedlichen vorbestimmten Positionen um den Patiententunnel angeordnet sind und einen dreidimensionalen Raum aufspannen, wobei die Sensoren (60) in Signalverbindung mit einer Ortungsvorrichtung (80) stehen, wobei das Verfahren die Schritte aufweist:
(S20) Erzeugen eines kodierten Ortungssignals mit dem Transponder (92);
(S30) Emittieren des kodierten Ortungssignals mit dem Transponder (92);
(S40) Empfangen des Ortungssignals mit der Mehrzahl der Sensoren (60);
(S50) Trennen des Ortungssignals von weiteren durch die Sensoren empfangenen Signalen durch die Ortungsvorrichtung (80) ;
(S60) Ermitteln einer Relativposition des Transponders (92) relativ zu den Sensoren durch die Ortungsvorrichtung (80) mittels des von den Sensoren erfassten Ortungssignals und
einer darauf angewendeten Ortsbestimmungsrelation.

6. Verfahren nach Anspruch 5, wobei das Verfahren weiterhin die Schritte aufweist:
(S10) Positionieren des Transponders (92) an einer vorbestimmten Relativposition zu den Sensoren (60);
(S70) Kalibrieren der Ortsbestimmungsrelation mit Hilfe der vorbestimmten Relativposition und der ermittelten Relativposition.

7. Verfahren nach Anspruch 5 oder 6, wobei das Verfahren weiterhin die Schritte aufweist:
(S80) Erfassen einer Position des Instruments (90) mit dem Transponder (92) in einer Magnetresonanzaufnahme;
(S90) Kalibrieren der Magnetresonanzaufnahme in Abhängigkeit von der in der Magnetresonanzaufnahme erfassten Position des Transponders (92) und der in Schritt (S60) mit der Ortungsvorrichtung (80) erfassten Position.

8. Computerprogrammprodukt, welches ein Programm umfasst und direkt in einen Speicher einer programmierbaren Steuerung (23) eines Magnetresonanztomographen (1) nach Anspruch 3 ladbar ist, mit Programmmitteln, um alle Schritte des Verfahrens nach einem der Ansprüche 5 bis 7 auszuführen, wenn das Programm in der Steuerung (23) des Magnetresonanztomographen (1) ausgeführt wird.

9. Elektronisch lesbarer Datenträger mit darauf gespeicherten elektronisch lesbaren Steuerinformationen, welche derart ausgestaltet sind, dass sie bei Verwendung des Datenträgers in einer Steuerung (23) eines Magnetresonanztomographen (1) nach Anspruch 3 das Verfahren nach einem der Ansprüche 5 bis 7 durchführen.

## Claims

1. Instrument for a medical examination or intervention using a magnetic resonance tomography system (1), wherein the instrument (90) has at least one transponder (92) which is configured to emit a locator signal in a frequency range of the magnetic resonance tomography system (1),
wherein
the locator signal is encoded in order to distinguish it from a magnetic resonance signal, wherein the transponder (92) has an energy source (93) and an oscillator (94), wherein the oscillator (94) is configured to generate the locator signal, **characterised in that**
the transponder (92) has a modulator (95), the encoding is a modulation by means of a pseudorandom code, and the modulator (95) is configured to modulate the locator signal by means of the encoding.

2. Instrument according to claim 1, wherein the encoding is a frequency encoding in a frequency range of the magnetic resonance tomography system (1).

3. Magnetic resonance tomography system, wherein the magnetic resonance tomography system (1) has a plurality of sensors (60) for detecting a noise signal and a noise suppression device (70) for suppressing noise signals in magnetic resonance signals received by local coils,
**characterised in that**
the magnetic resonance tomography system has a location tracking device (80) which is configured to pick up a locator signal of a transponder (92) by means of the sensors (60) and use said signal to determine a relative position of the transponder (92) in relation to the sensors (60),
wherein the locator signal is encoded in order to distinguish it from a magnetic resonance signal and the location tracking device (80) of the magnetic resonance tomography system (1) is configured to separate the locator signal of the transponder (92) of an instrument (90) from the noise signals by means of the encoding.

4. System composed of an instrument for a medical examination or a medical intervention using a magnetic resonance tomography system (1), wherein the instrument (90) has at least one transponder (92), which is configured to emit a locator signal in a frequency range of the magnetic resonance tomography system (1),
wherein the locator signal is encoded to distinguish it from a magnetic resonance signal
and a magnetic resonance tomography system (1) according to claim 3,
wherein the transponder (92) is configured to generate an encoded locator signal or the
magnetic resonance tomography system (1) is configured to generate an encoded locator signal and transmit the same via a line or wirelessly from the magnetic resonance tomography system to the transponder
and the location tracking device (80) is configured to separate the locator signal of the transponder (92) from the noise signals by means of the encoding.

5. Method for determining the relative position of a medical instrument (90) with a magnetic resonance tomography system (1), wherein the instrument has a transponder (92) for transmitting a locator signal, the magnetic resonance tomography system (1) comprises a plurality of sensors (60) for detecting the locator signal, wherein the sensors (60) are arranged in different predetermined positions around the patient tunnel and cover a three-dimensional space, wherein the sensors (60) are in signal connection with a location tracking device (80), wherein the method has the steps of:
(S20) generating an encoded locator signal by means of the transponder (92);
(S30) emitting the encoded locator signal by means of the transponder (92);
(S40) receiving the locator signal by means of the plurality of sensors (60);
(S50) separating the locator signal from further signals received by the sensors by means of the location tracking device (80);
(S60) determining a relative position of the transponder (92) in relation to the sensors by the location tracking device (80) by means of the locator signal detected by the sensors and a location determination relation applied thereto.

6. Method according to claim 5, wherein the method further has the steps of:
(S10) positioning the transponder (92) at a predetermined relative position in relation to the sensors (60);
(S70) calibrating the location determination relation with the aid of the predetermined relative position and the determined relative position.

7. Method according to claim 5 or 6, wherein the method further comprises the steps of:
(S80) detecting a position of the instrument (90) in a magnetic resonance acquisition by means of the transponder (92) ;
(S90) calibrating the magnetic resonance acquisition as a function of the position of the transponder (92) detected in the magnetic resonance acquisition and the position detected in step (S60) by means of the location tracking device (80).

8. Computer program product which comprises a program and can be loaded directly into a memory of a programmable controller (23) of a magnetic resonance tomography system (1) according to claim 3, having program means for performing all the steps of the method according to one of claims 5 to 7 when the program is executed in the controller (23) of the magnetic resonance tomography system (1).

9. Electronically readable data medium on which electronically readable control information is stored which is embodied in such a way that when the data medium is used in a controller (23) of a magnetic resonance tomography system (1) according to claim 3, the control information performs the method according to one of claims 5 to 7.

## Revendications

1. Instrument d'examen ou d'intervention médical en utilisant un tomodensitomètre (1) à résonance magnétique, l'instrument (90) ayant au moins un transpondeur (92), qui est conçu pour émettre un signal de localisation dans un domaine de fréquence du tomodensitomètre (1) à résonance magnétique,
dans lequel
le signal de localisation a un codage pour le distinguer d'un signal de résonance magnétique,
dans lequel le transpondeur (92) a une source (93) d'énergie et un oscillateur (94), dans lequel l'oscillateur (94) est conçu pour produire le signal de localisation,
**caractérisé en ce que**
le transpondeur (92) a un modulateur (95), le codage est une modulation par un code pseudo-aléatoire et le modulateur (95) est conçu pour moduler le signal de localisation par le codage.

2. Instrument suivant la revendication 1, dans lequel le codage est un codage de fréquence dans un domaine de fréquence du tomodensitomètre (1) à résonance magnétique.

3. Tomodensitomètre à résonance magnétique, dans lequel le tomodensitomètre (1) à résonance magnétique a une pluralité de capteurs (60) de détection d'un signal parasite et un dispositif (70) de déparasitage pour supprimer des signaux parasites de signaux de résonance magnétique reçus par des bobines locales,
**caractérisé en ce que**
le tomodensitomètre à résonance magnétique a un dispositif (80) de localisation, qui est conçu pour enregistrer, par les capteurs (60), un signal de localisation d'un transpondeur (92) et pour déterminer ainsi une position relative du transpondeur, (92) par rapport aux capteurs (60),
dans lequel
le signal de localisation a un codage pour le distinguer d'un signal de résonance magnétique et le dispositif (80) de localisation du tomodensitomètre (1) à résonance magnétique est conçu pour, au moyen du codage, séparer des signaux parasites le signal de localisation du transpondeur (92) d'un instrument (90), au moyen.

4. Système composé d'un instrument d'examen médical ou d'intervention médicale, en utilisant un tomodensitomètre (1) à résonance magnétique, dans lequel l'instrument (90) a au moins un transpondeur (92), qui est conçu pour émettre un signal de localisation dans un domaine de fréquence du tomodensitomètre (1) à résonance magnétique,
dans lequel le signal de localisation a un codage pour le distinguer d'un signal de résonance magnétique,
et d'un tomodensitomètre (1) à résonance magnétique suivant la revendication 3,
dans lequel le transpondeur (92) est conçu, pour produire un signal de localisation par un codage ou le tomodensitomètre (1) à résonance magnétique est conçu pour produire un signal de localisation par un codage et pour le transmettre par une ligne ou sans fil du tomodensitomètre à résonance magnétique au transpondeur
et le dispositif (80) de localisation est conçu pour séparer, au moyen du codage, le signal de localisation du transpondeur (92) de signaux parasites, au moyen du codage.

5. Procédé de détermination de la position relative d'un instrument (90) médical par un tomodensitomètre (1) à résonance magnétique, dans lequel l'instrument a un transpondeur (92) d'émission d'un signal de localisation, le tomodensitomètre (1) à résonance magnétique a une pluralité de capteurs (60) de détection du signal de localisation, dans lequel les capteurs (60) sont disposés en des positions différentes déterminées à l'avance autour du tunnel du patient et sous-tendent un espace à trois dimensions, dans lequel les capteurs (60) sont en liaison du signal avec un dispositif (80) de localisation,
dans lequel le procédé a les stades :
(S20) production d'un signal codé de localisation par le transpondeur (92) ;
(S30) émission du signal codé de localisation par le transpondeur (92) ;
(S40) réception du signal de localisation par la pluralité des capteurs (60) ;
(S50) séparation par le dispositif (80) de localisation du signal de localisation d'autres signaux reçus par les capteurs ;
(S60) détermination par le dispositif (80) de localisation d'une position relative du transpondeur (92) par rapport aux capteurs, au moyen du signal de localisation détecté par les capteurs et d'une relation de détermination de localisation, qui y est appliquée.

6. Procédé suivant la revendication 5, dans lequel le procédé comporte en outre des stades :
(S10) mise en position du transpondeur (92) en une position relative déterminée à l'avance par rapport aux capteurs (60) ;
(S70) étalonnage de la relation de détermination de localisation à l'aide de la position relative déterminée à l'avance et de la position relative déterminée.

7. Procédé suivant la revendication 5 ou 6, dans lequel le procédé comporte en outre les stades :
(S80) détection d'une position d'instrument (90) par le transpondeur (92) dans un enregistrement de résonance magnétique ;
(S90) étalonnage de l'enregistrement de résonance magnétique en fonction de la position détectée dans l'enregistrement de résonance magnétique du transpondeur (92) et de la position détectée dans le stade (S60) par le dispositif (80) de localisation.

8. Produit de programme d'ordinateur, qui comprend un programme et qui peut être chargé directement dans la mémoire d'une commande (23) programmable d'un tomodensitomètre (1) à résonance magnétique suivant la revendication 3, comprenant des moyens de programme pour exécuter tous les stades du procédé suivant l'une des revendications 5 à 7, lorsque le programme est exécuté dans la commande (23) du tomodensitomètre (1) à résonance magnétique.

9. Support de données déchiffrable électroniquement ayant des informations de commande déchiffrables électroniquement, qui sont mises en mémoire et qui sont conformées, de manière à ce qu'elles exécutent, lors de l'utilisation du support de données dans une commande (23) de tomodensitomètre (1) à résonance magnétique suivant la revendication 3, le procédé suivant l'une des revendications 5 à 7.
